# EUROPEAN PATENT APPLICATION

(11) **EP 4 079 361 A1**
(43) Date of publication of application: **26.10.2022**
(21) Application number: 22158699.3
(22) Date of filing: 25.02.2022
(51) Int. Cl.: A61M 16/10, A61M 16/12

(54) **A METHOD OF CONTROLLING A HIGH FLOW OXYGEN DEVICE**

(30) Priority: 21.04.2021 TR 202106951
(71) Applicant: Arçelik Anonim Sirketi, 34445 Istanbul (TR)
(72) Inventor: DINCER, Mehmet Onur, 34445 ISTANBUL (TR); KERPICCI, Husnu, 34445 ISTANBUL (TR); KANDEMIR, Nihat, 34445 ISTANBUL (TR)

(57) **Abstract**

The present invention relates to a method of controlling the operation of a high flow oxygen device (1) comprising the steps of; providing a casing enclosing the operational parts of the high flow oxygen device (1), providing a gas flow block (2) having a first inlet and a second inlet for introducing oxygen and ambient air into the gas flow block (2) respectively, providing a discharge valve (3) for discharging ambient air inside the casing to an outer surface of the high flow oxygen device (1) once activated, providing a blower (4) for introducing ambient air into the gas flow block (2) via the second inlet, providing a temperature sensor (5) configured to detect the temperature of the air inside the high flow oxygen device (1), providing a control unit in connection with the blower (4), the temperature sensor (5) and the discharge valve (3).

## Description

The present invention relates to a high flow oxygen device, in particular to a method of controlling a high flow oxygen device.

High flow oxygen devices are used for supporting patients having difficulty in breathing. High flow oxygen devices generally use an inlet for oxygen and another inlet for introducing ambient air into a mixing chamber. After mixing oxygen and ambient air, the mixture is transmitted to a patient. In some cases, the patient may require %90 or a higher percentage of oxygen to sustain the patient. If the required gas is mainly oxygen, the ambient air is not sucked into the high flow oxygen device. During long operation hours of high flow oxygen device, inner temperature of the high flow oxygen device increases. This causes various problems such as the reduction of the operational efficiency of the high flow oxygen device. Another problem caused by the increased temperature is that the hot air is sucked via the blower and transmitted to the patient. Also temperature may damage the operational parts of the high flow oxygen device, thus causing economical loss.

A prior art publication in the technical field of the present invention may be referred to as US2017002830A1 among others, the document disclosing a blower for a breathing apparatus having a diffuser for increasing static pressure and/or reducing noise and/or mitigating pressure instabilities and/or managing reverse flow.

A prior art publication in the technical field of the present invention may be referred to as US2019307978A1 among others, wherein the document discloses control methods that can indirectly determine incorrect connections between components in a respiratory therapy system.

A prior art publication in the technical field of the present invention may be referred to as WO2020204731A1 among others, wherein various control methods can indirectly determine incorrect connections between components in a respiratory therapy system.

A prior art publication in the technical field of the present invention may be referred to as US2007181127 among others, wherein a method for judging the reverse connection of a flow sensor is disclosed.

An objective of the present invention is to provide a high flow oxygen device having improved safety.

Another objective of the present invention is to provide a high flow oxygen device having temperature control.

The method realized to achieve the aim of the present invention and disclosed in the first claim comprises a method of controlling of a high flow oxygen device. The method comprises various steps. First of all, a casing is provided wherein the operational parts of the high flow oxygen device are enclosed. Additionally, a gas flow block is provided inside the casing. The gas flow block is provided with a first inlet and a second inlet wherein said inlets are used for introducing oxygen and ambient air into the gas flow block respectively. A discharge valve is provided on the gas flow block wherein the discharge valve is used for discharging ambient air entering the gas flow block when and if activated. A blower is provided inside the casing of the high flow oxygen device and is used to transmit ambient air into the gas flow block via a second inlet. The high flow oxygen device further comprises a temperature sensor. The temperature sensor continuously measures the temperature of the ambient air inside the casing. In order to control aforementioned parts, a control unit is provided. The control unit is in connection with the blower, temperature sensor and the discharge valve. The connection may be achieved wirelessly or by means of wires extending between the control unit and the said parts. The control unit receives the temperature information regarding the ambient air inside the casing via the temperature sensor and activates the discharge valve to discharge ambient air when and if the temperature reading provided by means of the temperature sensor exceeds a predetermined temperature. By means of this, high flow oxygen device can be cooled down. Another advantageous effect provided by means of the control unit and the temperature sensor is that the ambient air and oxygen mixture transmitted to a patient has an optimal temperature.

By means of the present invention, overheating of the high flow oxygen device is prevented thanks to the discharge valve and the control unit.

Another advantageous effect provided by means of the invention is that the gaseous mixture transmitted to the patient has an appropriate temperature thus preventing possible health problems.

The drawings are not meant to delimit the scope of protection as identified in the claims nor should they be referred to alone in an effort to interpret the scope identified in the claims without recourse to the technical disclosure in the description of the present invention.

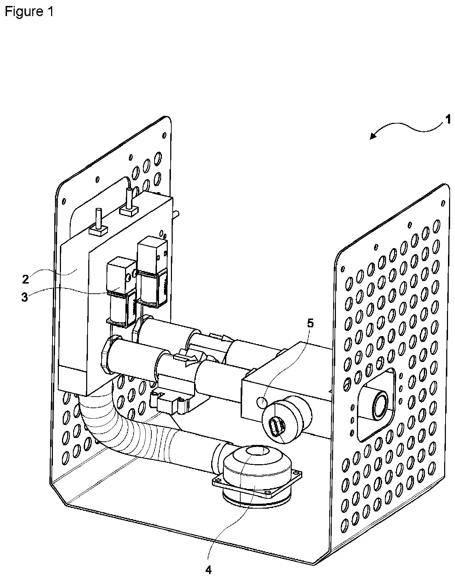

Figure 1 - is a perspective view of the high flow oxygen device

The following numerals are assigned to different parts demonstrated in the drawings and referred to in the present detailed description of the invention:
1. High flow oxygen device
2. Gas flow block
3. Discharge valve
4. Blower
5. Temperature sensor

The present invention relates to a method of controlling the operation of a high flow oxygen device (1) comprising the steps of; providing a casing enclosing the operational parts of the high flow oxygen device (1), providing a gas flow block (2) having a first inlet and a second inlet for introducing oxygen and ambient air into the gas flow block (2) respectively, providing a discharge valve (3) for discharging ambient air inside the casing to an outer surface of the high flow oxygen device (1) once activated, providing a blower (4) for introducing ambient air into the gas flow block (2) via the second inlet, providing a temperature sensor (5) configured to detect the temperature of the air inside the high flow oxygen device (1), providing a control unit in connection with the blower (4), the temperature sensor (5) and the discharge valve (3).

The present invention further involves the step wherein the control unit activates the discharge valve (3) and the blower (4) to discharge the ambient air inside the casing if the temperature of ambient air inside the casing exceeds a predetermined temperature.

The present invention discloses the method of operation of the high flow oxygen device (1). The gas flow block (2) is provided with the first inlet and the second inlet wherein said inlets are used for introducing oxygen and ambient air into the gas flow block (2) respectively. The discharge valve (3) is provided on the gas flow block (2) and is configured to discharge ambient air entering the gas flow block (2) to an outer surface of the high flow oxygen device (1) when and if activated. The blower (4) is provided inside the casing of the high flow oxygen device (1) and is configured to provide the gas flow block (2) with ambient air. The blower (4) sucks in ambient air that is inside the casing and forces ambient air to flow towards the gas flow block (2). The temperature sensor (5) is provided inside the casing of the gas flow block (2) and measures the temperature of the ambient air inside the casing on predetermined time intervals. The high flow oxygen device (1) is further provided with the control unit wherein the control unit controls the operational functions of the high flow oxygen device (1). The control unit is connected to the blower (4), the temperature sensor (5) and the discharge valve (3). The control unit may activate or deactivate the blower (4). Similarly, the control unit may activate or deactivate the discharge valve (3) to discharge some of the ambient air inside the casing to the outer surface of the high flow oxygen device (1). The control unit is also connected to the temperature sensor (5) and measures the temperature of the ambient air inside the casing. The connection between the control unit and the said parts can be achieved wirelessly or by means of electrical cables. The control unit is configured to detect the temperature of the ambient air via the temperature sensor (5). In case, wherein the temperature of the ambient air and therefore that of the inner volume of the casing exceeds a predetermined temperature, the control unit activates the discharge valve (3) to discharge some of the hot ambient air inside the casing, ergo cooling the high flow oxygen device (1). By means of this invention, the temperature of the high flow oxygen device (1) can be controlled effectively. As a result of this, it is ensured that the oxygen and ambient air mixture transmitted to a patient respiratory circuit is cooled. Another advantageous effect provided by means of this invention is that the temperature of the high flow oxygen device (1) is controlled which in turn will increase operational efficiency and life time of the high flow oxygen device (1).

An advantageous effect provided by means of the invention is that the high flow oxygen device (1) is prevented from overheating by means of the control unit that is configured to sense the inner temperature of the casing by means of the temperature sensor (5). The control unit, upon detecting a rise in the temperature, activates the discharge valve (3) and the blower (4) to discharge the ambient air inside the casing if the temperature of ambient air inside the casing exceeds a predetermined temperature.

Another advantageous effect provided by means of the invention is that the ambient air and oxygen mixture transmitted to a patient respiratory circuit is cooled.

## Claims

1. A method of controlling the operation of a high flow oxygen device (1) comprising the steps of;
providing a casing enclosing the operational parts of the high flow oxygen device (1),
providing a gas flow block (2) having a first inlet and a second inlet for introducing oxygen and ambient air into the gas flow block (2) respectively, providing a discharge valve (3) for discharging ambient air inside the casing to an outer surface of the high flow oxygen device (1) once activated,
providing a blower (4) for introducing ambient air into the gas flow block (2) via the second inlet,
providing a temperature sensor (5) configured to detect the temperature of the air inside the high flow oxygen device (1),
providing a control unit in connection with the blower (4), the temperature sensor (5) and the discharge valve (3),
wherein the control unit activates the discharge valve (3) and the blower (4) to discharge the ambient air inside the casing if the temperature of ambient air inside the casing exceeds a predetermined temperature.
